(19) Europäisches Patentamt
European Patent Office
Office européen des brevets

(11) **EP 1 176 131 B1**

(12) **EUROPÄISCHE PATENTSCHRIFT**

(45) Veröffentlichungstag und Bekanntmachung des Hinweises auf die Patenterteilung:
**09.03.2005 Patentblatt 2005/10**

(51) Int Cl.[7]: **C07C 31/26**, C07C 29/76, C07C 29/141

(21) Anmeldenummer: **01117446.3**

(22) Anmeldetag: **19.07.2001**

(54) **Verfahren zur Herstellung von Sorbitolen aus Standard-Glucose**

Method of preparation of sorbitols from standard-glucose

Procédé de préparation de sorbitols à partir de standard-glucose

(84) Benannte Vertragsstaaten:
**AT BE CH CY DE DK ES FI FR GB GR IE IT LI LU MC NL PT SE TR**

(30) Priorität: **24.07.2000 DE 10036189**

(43) Veröffentlichungstag der Anmeldung:
**30.01.2002 Patentblatt 2002/05**

(73) Patentinhaber: **DHW Deutsche Hydrierwerke GmbH Rodleben**
**06862 Rodleben (DE)**

(72) Erfinder:
• **Theoleyre, Marc-André**
**75020 Paris (FR)**
• **Konetzke, Gerhard, Dr.rer.nat. Dipl.-Chem.**
**06847 Dessau (DE)**

• **Schmidt, Klaus, Dr.rer.nat. Dipl.-Chem.**
**06862 Rodleben (DE)**
• **Weidemann, Reinhold, Dipl.-Ing.**
**14827 Wiesenburg (DE)**

(74) Vertreter: **Tragsdorf, Bodo, Dipl.-Ing.**
**Patentanwalt**
**Heinrich-Heine-Strasse 3**
**06844 Dessau (DE)**

(56) Entgegenhaltungen:
**EP-A- 0 168 315        DE-A- 19 612 826**
**US-A- 4 422 881        US-A- 5 127 957**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft ein Verfahren zur Herstellung von Sorbitolen durch Hydrierung von sogenannter Standardglucose (Zuckerspektrum 92 bis 96 % Dextrose, Di- und Oligosaccharide ad 100 %) mit anschließender Entsalzung und Eindampfung bis zu 70 %Trockensubstanz (TS) und nachfolgender chromatographischer Trennung des erhaltenen Gemisches von Zuckeralkoholen an stark sauren Kationenaustauschharzen.

Sorbitol wird wegen seiner besonderen chemischen, physikalisch-chemischen und physiologischen Eigenschaften in vielen Industriezweigen und zu sehr unterschiedlichen Zwecken verwendet (Nahrungsmittel, Pharmaka, Kosmetika, Rohstoff für chemische Synthesen, Hilfsmittel für verschiedene industrielle Anwendungsbereiche u.a.). Handelsüblich sind sowohl kristalline Produkte als auch konzentrierte Lösungen mit einem Trockensubstanzgehalt von meist 70% und einem Sorbitolgehalt von > 98,5 % bezogen auf Trockensubstanz (%-TS). Die einzelnen Anwendungsgebiete erfordern Sorbitole mit festgelegten Reinheitsgraden bzw. bestimmter Zusammensetzung aus verschiedenen Zuckeralkoholen.

Technisch werden alle genannten Sorbitol-Produkte durch Hydrierung von wäßrigen Zuckerlösungen entsprechender Zusammensetzung gewonnen, wobei die Monosaccharide zu Monosaccharidalkoholen (z.B. Glucose zu Sorbitol) und Oligosaccharide endständig zu Oligosaccharidalkoholen hydriert werden. In Abhängigkeit vom pH-Wert wird ein kleiner Anteil der Oligosaccharide während der Hydrierung hydrolysiert, so daß der Anteil an Monosaccharidalkoholen immer etwas höher ist als die Summe der Monosaccharide der Ausgangslösung. Je nach den Hydrierbedingungen beträgt der Restglucosegehalt im Allgemeinen 0,05 - 0,2 %-TS. Außerdem entstehen - in Abhängigkeit von den Prozeßbedingungen pH-Wert und Temperatur - durch Epimerisierung von Glucose geringe Mengen Mannitol (bis zu 1,5 %-TS) und Iditol (0,1 - 0,3 %-TS). Die im Ergebnis der Hydrierung erhaltenen wäßrigen Lösungen von Sorbitol bzw. Mischungen von Sorbitol mit anderen Zuckeralkoholen werden anschließend zur Abtrennung von Katalysatorpartikeln filtriert, mittels Ionenaustauschharzen entsalzt und bis zu einer Konzentration von 70 %TS eingedampft.

Zur technischen Herstellung von Sorbitol mit ca. 99 %iger Reinheit müssen also üblicherweise Glucoselösungen gleicher Reinheit eingesetzt werden.

Glucose wird heute nahezu ausschließlich durch enzymatische Hydrolyse von Stärke gewonnen. Die theoretisch erreichbare Glucosekonzentration beträgt maximal 97 %-TS (M.W. Kearsley und S.Z. Dziedzic; Handbook of Starch Hydrolysis Products and their Derivatives; S. 47 ff.; 1995). Das Hydrolysat ist die sogenannte Standard-Glucose mit einem Glucosegehalt von 92 - 96 %-TS. Die weitere Anreicherung von Glucose auf > 99 %-TS ist aufwendig und wird entweder durch Kristallisation (Ullmann, Vol. A 12, S. 467 ff.; 1989) oder durch chromatographische Abtrennung der Di- und Oligosaccharide von Dextrose an Kationenaustauschharzen erreicht (F.W. Schenck, Starch Hydrolysis Products: Worldwide Technology, Production and Applications, S. 568, New York 1992).

Bei gleichzeitiger Herstellung von Sorbitolen mit einer Reinheit von ca. 99 % einerseits und andererseits von nicht bzw. schwer kristallisierenden Sorbitollösungen unter technischen Bedingungen ist zur Vermeidung von Produktvermischungen der apparative Aufwand hoch. Außer der getrennten Lagerhaltung für die verschiedenen einzusetzenden Rohstoffe sind für die Verfahrensstufen Hydrierung, Filtration, Entsalzung und Eindampfung apparativ getrennte Produktionslinien oder im Fall des Produktwechsels in einer Anlage der zwischenzeitliche Durchsatz einer größeren Wassermenge als Puffer notwendig. Außerdem sind die jeweiligen Hydrier bedingungen (Temperatur, Durchsatz) an die unterschiedlichen Rohstoffe anzupassen. Dies ist mit negativen Auswirkungen auf Aktivität und Lebensdauer des Katalysators verbunden. Von Nachteil sind auch die erforderlichen Trenn- und Reinigungsstufen der Glucose, die die Wirtschaftlichkeit der Verfahren zur Herstellung von Sorbitolen beeinträchtigen.

Zur Herstellung hochreiner Sorbitole mit Gehalten an Di- und Oligosaccharidalkoholen max. 0,15 %-TS und Restglucose max. 0,02 %-TS ist zunächst der Einsatz entsprechend reiner Glucoselösungen Voraussetzung, der Restglucosegehalt nach der Hydrierung ist praktisch rohstoffunabhängig.

Aus der DE 196 12 826 C2 ist ein Verfahren zur Herstellung von Sorbitol mit einer Reinheit von mindestens 99,5 % bekannt, wobei aus einer hydrierten und entsalzten Sobitollösung normaler Qualität der überwiegende Teil der Nebenprodukte durch ein chromatographisches Trennverfahren unter Einhaltung spezieller Verfahrensbedingungen abgetrennt wird.

In der Praxis wird zur Herstellung großer Mengen von Sorbitol mit hohem Reinheitsgrad die chromatographische Trennmethode nicht bei den Zuckeralkoholen, sondern bereits bei der als Rohstoff eingesetzten Glucose angewendet.

Bei der chromatographischen Trennung von Zuckeralkoholen an stark sauren Kationenaustauschharzen ist zu berücksichtigen, daß die Retention der Zuckeralkohole deutlich höher ist als die der jeweils zugehörigen Zucker. Das hat zur Folge, daß zur Eluierung der Zuckeralkoholkomponenten größere Mengen Wasser notwendig sind und daher insbesondere bei der jeweiligen Extraktfraktion eine geringere TS-Konzentration zu erwarten ist. Andererseits sind die Retentionsunterschiede zwischen den Zuckeralkoholen größer als bei den entsprechenden Zuckern und können zur Lösung der Trennaufgabe ausgenutzt werden.

Als kontinuierlich arbeitendes chromatographisches Verfahren zur Trennung einer Stoffmischung in zwei Fraktionen ist bereits seit längerem das sogenannte SMB (Simulated-Moving-Bed) - Verfahren bekannt (US-PS 2,985,589).

Beim SMB-Verfahren werden eine beliebige Anzahl an Säulen zu einer endlosen Schleife hintereinandergeschaltet, wobei die Gesamtzahl ein Vielfaches von vier ist, da dieses System vier chromatographische Zonen besitzt. Das als Adsorbent eingesetzte Austauschharz wird zu jeweils gleichen Mengen auf die Säulen aufgeteilt. Innerhalb eines Zyklus werden eine Anzahl an Schritten durchgeführt, die der Anzahl der eingesetzten Säulen entspricht. Die erste Zone wird begrenzt zwischen Eluentzufluß und Extraktabfluß, die zweite Zone zwischen Extraktabfluß und Rohproduktzufluß, die dritte Zone zwischen Rohproduktzufluß und Raffinatabfluß und die vierte Zone zwischen Raffinatabfluß und Eluentzufluß. Nach einer definierten Zeit werden die Leitungen umgeschaltet, wodurch eine Bewegung des Säulenbettes in der entgegengesetzten Richtung simuliert wird.

Eine spezielle, erst seit kurzem bekannte Ausführungsform des SMB-Verfahrens ist der sogenannte Sequential Simulated Moving Bed Process (abgekürzt SSMB), bei dem jede chromatographische Zone bzw. jeder SMB-Schritt in 3 oder 4 sogenannte "Sequenzen" bzw. "Unterschritte" unterteilt ist (D. Paillat, M. Cotillon; "Starch Convention 1999" Detmold, Germany, S. 7).

In der EP-A-0 168 315 ist ein Verfahren zur chromatographischen Anreicherung von ca. 94%igem Sorbitol auf> 98 % (bezogen jeweils auf Trockensubstanz) nach dem SMB-Verfahren beschrieben, wodurch zu dessen Herstellung auf den Einsatz entsprechend reiner Dextrose, die entweder durch Kristallisation oder Chromatographie von Stärkehydrolysat gewonnen wird, verzichtet werden kann. Wesentliche Nachteile dieser chromatographischen Anreicherung von Sorbitol nach dem beschriebenen SMB-Verfahren sind die geringe Harzleistung sowie die sehr geringe Konzentration und die Zusammensetzung der Raffinatfraktion, die deshalb teilweise verworfen und zum anderen Teil erneut hydrolysiert, gereinigt, eingedampft und hydriert werden muss.

Der Erfindung liegt die Aufgabe zugrunde, ein verbessertes großtechnisches Verfahren zur Herstellung von Sorbitolen aus Standard-Glucose zu schaffen, mit dem es möglich ist, in besonders wirtschaftlicher Betriebsweise Sorbitol mit einer Reinheit von mindestens 99,5 %-TS, einem Gehalt an Gesamtzuckern von max. 0,15 %-TS und einem Restglucosegehalt von max. 0,02 %-TS zu erhalten.

Erfindungsgemäß wird die Aufgabe durch die im Anspruch 1 angegebenen Merkmale gelöst. Geeignete Ausgestaltungsvarianten sind Gegenstand der Ansprüche 2 bis 16.

Gemäß der vorgeschlagenen Verfahrensweise wird als Ausgangsprodukt Glucose mit einem Glucosegehalt von 92 bis 96 %-TS eingesetzt, die als Standard-Glucose bezeichnet wird. Nach der Hydrierung der Glucoselösung, Entsalzung und Eindampfung auf ca. 60 bis 70 %TS wird eine Lösung von Zuckeralkoholen erhalten, die zu ca. 95,5 %-TS aus Monosaccharidalkoholen (DP = 1) und zu ca. 4,5 %-TS aus Di- und Oligosaccharidalkoholen (DP $\geq$2) besteht, wobei DP "Degree of Polymerisation" bedeutet.

Der Restglucosegehalt beträgt 0,04 bis 0,10 %-TS. Der etwas höhere Gehalt an Monosaccharidalkoholen im hydrierten Produkt gegenüber dem Rohstoff ist auf partielle Hydrolyse der Bestandteile mit DP $\geq$2 während der bei einem pH-Wert von ca. 4 durchgeführten Hydrierung zurückzuführen und stellt einen positiven Nebeneffekt dar. Die Monosaccharidalkohole bestehen zu einem Anteil von > 99% aus Sorbitol, 0,3 bis 1,0 % aus Mannitol und 0,1 bis 0,3 % aus Iditol.

Die nachfolgende chromatographische Auftrennung des beschriebenen Gemisches von Zuckeralkoholen beruht auf der Zunahme der Affinität der Einzelkomponenten zu sauren Ionenaustauschharzen in nachstehender Reihenfolge: Zuckeralkohole (DP $\geq$3); Glucose; Maltitol (DP = 2); Mannitol; Sorbitol und Iditol (DP = 1).

Bei der chromatographischen Trennung in zwei Fraktionen sind demzufolge im Raffinat die Komponenten mit relativ geringer Affinität angereichert (Zuckeralkohole DP $\geq$2, Glucose, Mannitol) und im Extrakt die Komponenten Sorbitol und Iditol mit stärkerer Affinität. Als Eluent wird Wasser eingesetzt. Durch Wahl entsprechender Chromatographie-Bedingungen kann das Trennergebnis hinsichtlich Zusammensetzung der Fraktionen, Harzleistung und Elutionsmittelverbrauch (Wasser) beeinflußt werden. Überraschenderweise wurde festgestellt, daß der Einsatz des SSMB-Vefahrens zur chromatographischen Trennung des Zuckeralkoholgemisches aus hydrierter Standard-Glucose unter Einhaltung ganz spezieller Verfahrensbedingungen zu einer Vielzahl an Vorteilen führt und eine neue großtechnisch umsetzbare Technologie darstellt, die als Einstrang-Prozeß zur Herstellung unterschiedlicher Sorbitolqualitäten über die Stufen Hydrierung - Entsalzung - Eindampfung - Trennung nach dem SSMB-Verfahren realisiert werden kann.

im Vergleich zu den bisher bekannten Verfahrensweisen zur Herstellung von hochreinem Sorbitol bietet das neue Verfahren folgende Vorteile:

- Herstellung aller genannten Sorbitolprodukte aus dem preiswerten Rohstoff Standard-Glucose.
- Möglichst große Rückhaltung des Sorbitols (Sorbitolmenge in der Extraktfraktion im Verhältnis zur Sorbitolmenge im Rohprodukt).
- Möglichst hohe TS-Konzentrationen der Extrakte und Raffinate.
- Möglichst hohe Kapazität der für den chromatographischen Prozeß eingesetzten Harze (angegeben in gTS/l Harz·Tag).

Der Verfahrensablauf der chromatographischen Trennung des Zuckeralkoholgemisches nach dem SSMB-Prozeß soll im folgenden an Hand von zwei Abbildungen erläutert werden, die folgendes zeigen:

Abb. 1    eine schematische Darstellung der vier chromatographischen Zonen und deren Definition durch die Ein- und Ausgänge der verschiedenen Flüssigkeiten, wobei unter a) der Ablauf des "ersten Schrittes" und unter b) der Ablauf des "zweiten Schrittes" nach der ersten Umschaltung dargestellt sind und

Abb. 2    eine schematische Darstellung der Aufteilung des "ersten Schrittes" in vier "Sequenzen".

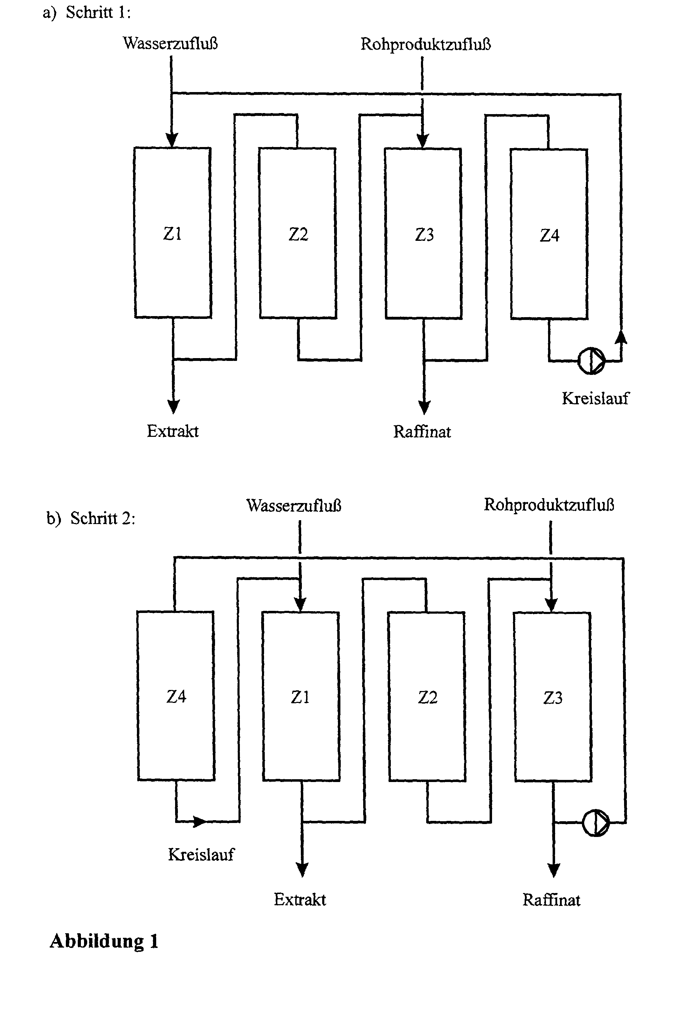

Als Adsorbent wird vorzugsweise ein stark saures Kationenaustauscherharz in der Ca-Form (Hersteller z.B. Fa. Dow oder Fa. Rohm & Haas) für Chromatographieanlagen eingesetzt. Das Harz ist auf mindestens vier, bevorzugterweise sechs oder acht, identischen Zellen verteilt. Typischerweise besteht das System aus vier Säulen, wobei jede Säule aus zwei identischen Zellen mit einer Harzbetthöhe von 2 m besteht. Die Unterteilung in Zellen ist in den gezeigten Abbildungen nicht dargestellt.
Die Position der Zu- und Abflüsse definiert die vier Zonen der Gesamtapparatur wie folgt (Abb. 1):

Zone 1 (Z1):    zwischen Wasserzufluß und Extraktabfluß
Zone 2 (Z2):    zwischen Extraktabfluß und Rohproduktzufluß
Zone 3 (Z3):    zwischen Rohproduktzufluß und Raffinatabfluß
Zone 4 (Z4):    zwischen Raffinatabfluß und Wasserzufluß

Während eines bestimmten Schrittes (z.B. Schritt 1 in Abb. 1) werden folgende spezifischen Flüssigkeitsströme durch die unterschiedlichen Zonen geleitet:

Z1:    Flüssigkeitsstrom Kreislauf plus Wasserzufluß
Z2:    Flüssigkeitsstrom Z1 minus Abfluß Extrakt
Z3:    Flüssigkeitsstrom Z2 plus Rohproduktzufluß
Z4:    Flüssigkeitsstrom Kreislauf

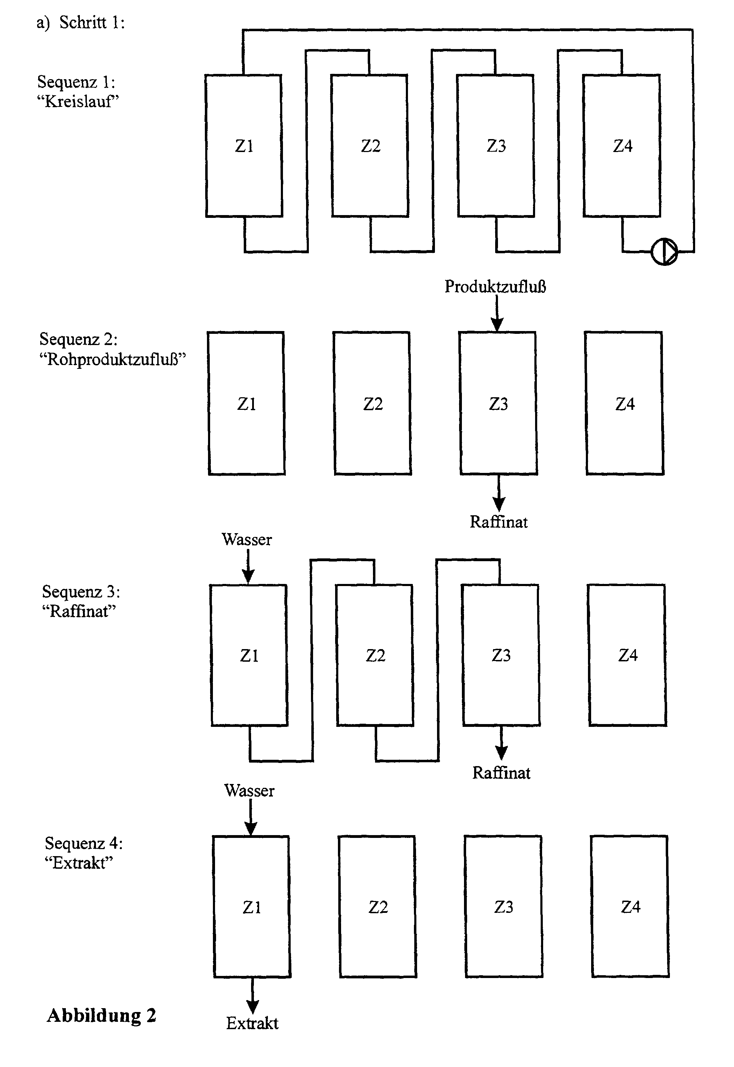

Am Ende eines Schrittes werden die Positionen der Zu- und Abflüsse um je eine Säule in Richtung des Flüssigkeitskreislaufes verschoben, wodurch die Bewegung des Harzes gegen den Flüssigkeitsstrom simuliert wird (Abb. 1, Schritt 2). Die Anzahl der Schritte pro Zyklus entspricht der Anzahl der verwendeten Säulen, wobei die einzelnen Schritte zeitlich gleichlang sind.
Die Komponenten mit geringerer Affinität zum Harz wandern mit der Flüssigphase zur nächsten Säule (bzw. Zone). Auf diese Weise gelangen diese Komponenten mit jedem Schritt zum Raffinatabfluß. Die Komponenten mit größerer Affinität zum Harz verbleiben nach dem Schrittwechsel am Adsorbent.
Jeder der vier Schritte ist außerdem in vier sogenannte Sequenzen unterteilt, wie in Abb. 2 an Hand des "Schrittes 1" gezeigt ist. Innerhalb eines jeden Schrittes finden somit diese vier Sequenzen statt, wobei die einzelnen Sequenzen der jeweiligen Schritte folgende Bedeutung haben:

Sequenz 1 ("Kreislauf"):

Die Flüssigphase zirkuliert im geschlossenem Kreislauf durch das gesamte System von Z4 nach Z1. Das bewirkt die Stoffwanderung im Harzbett.

Sequenz 2 ("Rohproduktzufluß"):

Der Rohproduktzufluß erfolgt am Anfang von Z3, das Rohprodukt eluiert eine Raffinatfraktion, die am Ende von Z3 aufgefangen wird.

Sequenz 3 ("Raffinat"):

Der Wasserzufluß erfolgt am Anfang von Z1 und eluiert eine weitere Raffinatfraktion, die am Ende von Z3 aufgefangen wird. Die Raffinatfraktionen der Sequenzen 2 und 3 werden als Gesamtfraktion aufgefangen.

Sequenz 4 ("Extrakt"):

Der Wasserzufluß erfolgt am Anfang von Z1 und eluiert die am Ende von Z1 aufgefangene Extraktfraktion.
Wie in Abb. 2 gezeigt, ist es möglich, die Sequenzen 2 und 4 gleichzeitig durchzuführen.
Auf diese Weise werden während jeder Sequenz genau definierte Volumina von Kreislaufprodukt, Rohprodukt und Wasser durchgesetzt.

Wichtigste Parameter für die chromatographische Trennung sind die sogenannten (Harz-)Bett-Volumina (BV1 bis BV4) in jeder Zone bzw. Säule, die als dimensionslose Größe wie folgt definiert sind:

$$BV = \frac{\text{Durchflußmenge x Sequenzzeit}}{\text{Harz-Volumen}},$$

wobei die Durchflußmenge die Durchflußrate von Eluent, Rohprodukt und Kreislaufprodukt als Volumen pro Zeiteinheit bedeutet und Harz-Volumen das Volumen pro Säule bzw. Zelle, wenn eine Säule aus mehreren Zellen besteht.

Wie in Abb. 2 gezeigt, setzen sich die Bett-Volumina der Zonen 1 bis 4 aus den Bett-Volumina der Sequenzen wie folgt zusammen:

Zone 1:   BV1 = BV (Sequenz 1) + BV (Sequenz 3) + BV (Sequenz 4)
Zone 2:   BV2 = BV (Sequenz 1) + BV (Sequenz 3)
Zone 3:   BV3 = BV (Sequenz 1) + BV (Sequenz 2) + BV (Sequenz 3)
Zone 4:   BV4 = BV (Sequenz 1)

Durch Variation von Durchflußmengen und Sequenzzeiten werden die Bett-Volumina der Sequenzen pro Zelle (und daraus sich ergebend die Bett-Volumina pro Zelle der Zonen) ermittelt, die sich besonders vorteilhaft auf das Trennergebnis auswirken

Bei gegebener Säulenabmessung können zur Beeinflussung des Trennergebnisses hinsichtlich Leistung und Zusammensetzung von Extrakt und Raffinat sowie unter Berücksichtigung unterschiedlicher Sorbitolgehalte im Rohprodukt (92 bis 97 %-TS) die Sequenzzeiten sowie die Durchflußraten (Wasser, Rohprodukt, Kreislauf) variiert werden, wobei bei einer Erhöhung der Durchflußraten der bei maximaler Strömungsgeschwindigkeit in Zone 3 auftretende maximale Druckverlust beim Übergang von Sequenz 2 auf Sequenz 3 zu berücksichtigen ist. Letzterer sollte aus technischen Gründen 1,5 bar/m nicht übersteigen. Die Durchführung der chromatographischen Trennung bei Temperaturen > 80 °C ermöglicht Fließgeschwindigkeiten bis zu 7 m/h sowie TS-Gehalte im Rohprodukt bis zu 70 %.

Die Zusammensetzung der im Ergebnis der Chromatographie erhaltenen Raffinatfraktion entspricht handelsüblichen nicht kristallisierenden Sorbitollösungen, während die Extraktfraktion je nach gewählter Länge der Zeitdauer der Sequenz 2 "Rohproduktzufluß" und der Sequenz 2 "Raffinat" entweder Sorbitol mit > 98.5% iger Reinheit oder hochreines Sorbitol (Sorbitolgehalt > 99,5 %-TS) mit sehr geringem Restglucosegehalt ergibt.

Unter den vorgenannten Betriebsbedingungen wird eine Harzleistung von bis zu 2,2 kg TS/l Harz-Tag erreicht.

Im Anschluß an die chromatographische Trennung werden die Fraktionen auf Lösungen mit je 70 %TS eingedampft. Die bei den Eindampfungsprozessen angefallenen Kondensate decken den Bedarf für das bei der Chromatographie als Eluent benötigte Wasser (ca. 5 l/kg TS).

Damit ist trotz großer Trennleistung der Wasserbedarf relativ gering und die Harzleistung von ca. 2 kg TS/l Harz·Tag relativ hoch, wodurch die Wirtschaftlichkeit der neuen Verfahrensweise wesentlich verbessert wird.

Die Sorbitol-Rückhaltung (Sorbitolanteil im Extrakt bezogen auf Sorbitolanteil des Einsatzproduktes) beträgt 75 bis 83 %. Eine größere Rückhaltung (bei weiterer Anreicherung der DP ≥ 2 - Zuckeralkohole im Raffinat) ist theoretisch und unter modifizierten Verfahrensbedingungen auch praktisch möglich, jedoch nicht sinnvoll, weil handelsübliche nichtkristallisierende 70 %ige Sorbitollösungen einen Sorbitolgehalt von mind. 75 %-TS verlangen.

Zusammenfassend ist festzustellen, daß im Gegensatz zum bekannten Stand der Technik nach dem erfindungsgemäßen Verfahren die durch Stärkehydrolyse zugängliche Standard-Glucose ohne sonst übliche Trenn- oder Reinigungsstufen zur Herstellung von Sorbitolen eingesetzt werden kann und daß über einen Einstrang-Prozeß (Hydrierung-Entsalzung-Eindampfung mit nachfolgender chromatographischer Trennung eines Gemisches von Zuckeralkoholen nach dem SSMB-Prozeß) in wirtschaftlich verbesserter Weise einerseits Sorbitol in gewünschter hoher Reinheit und andererseits nicht kristallisierendes Sorbitol als Zielprodukte erhalten werden.

Die Erfindung wird nachstehend an zwei Beispielen erläutert.

Beispiele 1 und 2

Verwendete Apparatur:

Für die Durchführung der chromatographischen Trennung nach dem SSMB-Prinzip wurde eine Pilotanlage mit 8 zylindrischen Behältern (Länge jeweils 1,35 m und Durchmesser 6 cm) verwendet. Jeder dieser Behälter enthielt als Kationenaustauscherharz 3,81 l stark saures Polystyrolharz in der Calciumform ( Dowex Mono 99 CA 320) mit der für chromatographische Trennungen im technischen Maßstab üblichen Korngrößenverteilung. Jeweils zwei hintereinandergeschaltete Behälter (Zellen) gehören zu einer Zone. Die Ansteuerung der Ventile in den Zu- und Abflußleitungen sowie der Kreisläufe zur Realisierung der Schritte und deren Unterteilung in 4 Sequenzen erfolgt nach den bei SSMB-Prozessen bekannten Regelungen.

Eingesetztes Rohprodukt:

Standard-Glucose mit einem Glucosegehalt von 95,3 % (bez. auf TS) wurde unter üblichen Bedingungen hydriert, entsalzt und eingedampft. Das auf diese Weise erhaltene Gemisch von Zuckeralkoholen wies folgende Kennzahlen auf:

| | |
|---|---|
| Dichte bei 20 °C = 1,27 g/m$^3$ | HPLC:DP $\geq$ 2 = 4,0 % |
| Trockensubstanz = 65,2 % | Mannitol = 0,6 % |
| Restglucose = 0,07 %-TS[1] | Sorbitol = 95,3 % |
| | Iditol = 0,1 % |

[1] *Analysenmethode: DHW-A 1736*

Prozeßbedingungen:

Die Beispiele 1 und 2 unterscheiden sich lediglich im Prozeßparameter Bett-Volumina. Im Vergleich zu Beispiel 1 wurde im Rahmen der Betriebsweise gemäß Beispiel 2 die Zeitdauer für die Sequenz 2 "Rohproduktzufluß" von 265 Sekunden auf 294 Sekunden verlängert und die Zeitdauer für die Sequenz 3 "Raffinat" von 180 Sekunden auf 154 Sekunden verkürzt, wodurch sich für die Zonen 1 bis 3 folgende angegebene Bettvolumina ergeben haben:

| | Beispiel 1 | Beispiel 2 |
|---|---|---|
| Fließraten: Kreislauf | 18 l/h | 18 l/h |
| Wasserzufluß | 18 l/h | 18 l/h |
| Rohproduktzufluß | 8,5 l/h | 8,5 l/h |
| Sequenzzeiten: | | |
| Sequenz 1 ("Kreislauf") | 300 sec. | 300 sec. |
| Sequenz 2 ("Rohproduktzufluß") | 265 sec. | 294 sec. |
| Sequenz 3 ("Raffinat") | 180 sec. | 154 sec. |
| Sequenz 4 ("Extrakt") | 265 sec. | 265 sec. |
| Gesamtschrittzeit | 12,4 min. | 12,5 min |
| Bett-Volumina pro Zelle: BV1 | 0,978 | 0,946 |
| BV2 | 0,628 | 0,596 |
| BV3 | 0,794 | 0,778 |
| BV4 | 0,392 | 0,392 |
| max. Strömungsgeschwindigkeit | 6,4 m/h | 6,4 m/h |
| Temperatur | 85°C | 85°C |
| max. Druckverlust (in Zone 3) | 1,5 bar/m | 1,5 bar/m |

Die Gesamtschrittzeit ergibt sich aus den vorgenannten Sequenzzeiten unter Berücksichtigung, daß jeweils die Sequenzen 2 und 4 gleichzeitig stattfinden.

Ergebnisse:

Die chromatographische Trennung ergab folgende Fraktionen:

| | Beispiel 1 | | Beispiel 2 | |
|---|---|---|---|---|
| | Raffinat (%) | Extrakt (%) | Raffinat (%) | Extrakt (%) |
| TS-Konzentration gem. n$_D^{20}$ | 6,8 | 28,0 | 6,7 | 30,7 |
| Gesamtzucker bez. auf TS[1] | 10,3 | 0,14 | 11,6 | 0,40 |

[1] *Analysenmethode: DHWN-A 1736*

(fortgesetzt)

|  | Beispiel 1 | | Beispiel 2 | |
|---|---|---|---|---|
|  | Raffinat (%) | Extrakt (%) | Raffinat (%) | Extrakt (%) |
| Restglucose bez. auf TS[1] | 0,20 | 0,01 | 0,14 | 0,03 |
| HPLC: DP $\geq$ 2 | 18,1 | 0,2 | 20,4 | 0,5 |
| Mannitol | 1,5 | 0,1 | 0,9 | 0,5 |
| Sorbitol | 80,4 | 99,6 | 78,7 | 98,9 |
| Iditol | < 0,05 | 0,1 | < 0,05 | 0,1 |

[1] *Analysenmethode: DHWN-A 1736*

Nach Eindampfen der Fraktionen auf 70 %TS wurden direkt die handelsüblichen Produkte hochreines Sorbitol (99,6 % Sorbitol / Extrakt Beispiel 1) bzw. reines Sorbitol (98,9 % Sorbitol / Extrakt Beispiel 2) sowie nichtkristallisierendes Sorbitol (Raffinate der Beispiele 1 und 2) erhalten.

Hinsichtlich der Rückhaltung von Sorbitol im Extrakt, des Eluent-Verbrauches sowie der Harzleistung wurden folgende Ergebnisse erzielt:

|  | Beispiel 1 | Beispiel 2 |
|---|---|---|
| Rückhaltung von Sorbitol im Extrakt | 81,9 % | 82,5 % |
| Wasserverbrauch (Eluent) ($IH_2O$/kgTS) | 5,1 | 4,6 |
| Harzleistung         (kgTS/IHarz x Tag) | 1,92 | 2,12 |

## Patentansprüche

**1.**    Verfahren zur Herstellung von Sorbitolen durch Hydrierung von Glucose mit einem Glucosegehalt von 92 bis 96 %-Trockensubstanz (TS), Entsalzung und Eindampfung auf bis zu 70 % TS sowie anschließende chromatographische Trennung des als Rohprodukt erhaltenen Zuckeralkohol-Gemisches nach dem SSMB (Sequential Simulated Moving Bed) - Verfahren an einem stark sauren Kationenaustauschharz, wobei jeder Zyklus aus einer der Anzahl der chromatographischen Zonen entsprechenden Anzahl von Schritten besteht und jeder Schritt in vier Sequenzen unterteilt wird, in eine erste Sequenz 1 "Kreislauf", eine zweite Sequenz 2 "Rohproduktzufluss", eine dritte Sequenz 3 "Raffinat" und eine vierte Sequenz 4 "Extrakt", und innerhalb eines Schrittes die Sequenzen 2 und 4 gleichzeitig durchgeführt und die Raffinatfraktionen der Sequenzen 2 und 3 als Gesamtfraktion, die eine handelsübliche nicht kristallisierende Sorbitollösung ist, aufgefangen werden, und zur Beeinflussung des Trennergebnisses hinsichtlich der Harzleistung, der Zusammensetzung von Extrakt und Raffinat und des Sorbitolgehaltes der Extraktfraktion die Bett-Volumina der Säulen verändert werden, wobei durch Veränderung der Zeitdauer einer oder mehrerer Sequenzen und/oder der Durchflussrate unterschiedliche Extraktfraktionen als Sorbitol mit einem Sorbitolgehalt von mindestens 98,5 %-TS (reines Sorbitol) oder als Sorbitol mit einem Sorbitolgehalt von mindestens 99,5 %-TS (hochreines Sorbitol) erhalten werden, und abschließend die anfallenden Raffinat- und Extraktfraktionen auf Lösungen mit bis zu 70 %TS eingedampft werden.

**2.**    Verfahren nach Anspruch 1, **dadurch gekennzeichnet, dass** durch Veränderung der Zeitdauer der Sequenzen 2 und 3 und/oder der Durchflussraten die Bettvolumina der Zellen (in den Zonen) auf folgende Werte eingestellt werden:

BV1 = 0,94 - 0,96, BV2 = 0,58 - 0,60, BV3 = 0,76 - 0,78 und BV4 = 0,38 - 0,40, und als Extraktfraktion a) Sorbitol mit einem Sorbitolgehalt von mindestens 98,5 %-TS, einem Gehalt an Gesamtzuckern von max. 0,5 %-TS und einem Restglucosegehalt von max. 0,06 %-TS erhalten wird.

**3.**    Verfahren nach Anspruch 1, **dadurch gekennzeichnet, daß** durch Veränderung der Zeitdauer der Sequenzen 2 und 3 und/oder der Durchflußraten die Bettvolumina der Zellen (in den Zonen) auf folgende Werte eingestellt werden:

BV1 = 0,97 - 0,99, BV2 = 0,62 - 0,64, BV3 = 0,78 - 0,81 und BV4 = 0,38 - 0,40, und als Extraktfraktion b) hochreines Sorbitol mit einem Sorbitolgehalt von mindestens 99,5 %-TS, einem Gehalt an Gesamtzuckern von max. 0,15 %-TS und einem Restglucosegehalt von max. 0,02 %-TS erhalten wird.

4. Verfahren nach einem der Ansprüche 1 bis 3, **dadurch gekennzeichnet, daß** zur Umstellung der Qualität der Extraktfraktion von reinem auf hochreines Sorbitol die Zeitdauer der Sequenz 2 "Rohproduktzufluß" erhöht und die Zeitdauer der Sequenz 3 "Raffinat" verringert wird und bei einer Umstellung der Betriebsweise von hochreinem auf reines Sorbitol nur die Zeitdauer der Sequenz 2 "Rohproduktzufluß" verringert und die Zeitdauer der Sequenz 3 "Raffinat" ohne Änderung weiterer Verfahrensparameter erhöht wird.

5. Verfahren nach Anspruch 4, **dadurch gekennzeichnet, daß** zur Umstellung von der Extraktfraktion a) auf die Extraktfraktion b) die Zeitdauer der Sequenz 2 um ca. 11% erhöht und die der Sequenz 3 um ca. 15 % verringert wird und zur Umstellung von der Extraktfraktion b) auf die Extraktfraktion a) die Zeitdauer der Sequenz 2 um ca. 11 % verringert und die der Sequenz 3 um ca. 15 % erhöht wird.

6. Verfahren nach einem der Ansprüche 1 bis 5, **dadurch gekennzeichnet, daß** als Eluent Wasser in einer Menge von 5 bis 6 l/kg Trockensubstanz eingesetzt wird.

7. Verfahren nach einem der Ansprüche 1 bis 6, **dadurch gekennzeichnet, daß** die chromatographische Trennung bei einer Temperatur von bis zu 90 °C durchgeführt wird.

8. Verfahren nach Anspruch 7, **dadurch gekennzeichnet, daß** bei Einhaltung von Temperaturen oberhalb von 80 °C ein Rohprodukt mit TS-Konzentration von 65 bis 70 % eingesetzt wird und Fließgeschwindigkeiten bis zu 7 m/h erreicht werden.

9. Verfahren nach einem der Ansprüche 1 bis 8, **dadurch gekennzeichnet, daß** das eingesetzte Rohprodukt eine TS-Konzentration von 62 bis 70 %, vorzugsweise 65 % bis 67 %, aufweist.

10. Verfahren nach einem der Ansprüche 1 bis 9, **dadurch gekennzeichnet, daß** beim Übergang von der Sequenz 2 "Rohproduktzufluß" auf die Sequenz 3 "Raffinat" ein Druckverlust von maximal 1,5 bar/m eingehalten wird.

11. Verfahren nach einem der Ansprüche 1 bis 10, **dadurch gekennzeichnet, daß** die Sorbitol-Rückhaltung, d.h. der Sorbitolanteil im Extrakt bezogen auf den Sorbitolanteil des Rohproduktes, 75 bis 83 % beträgt.

12. Verfahren nach einem der Ansprüche 1 bis 11, **dadurch gekennzeichnet, daß** eine auf die Harzmenge bezogene Kapazität von bis zu 2200 g Trockensubstanz/l Harz-Tag erreicht wird.

13. Verfahren nach einem der Ansprüche 1 bis 12, **dadurch gekennzeichnet, dass** als Rohprodukt eine direkt aus der Stärkehydrolyse und ohne chromatographische oder anderweitige Anreicherung gewonnene Glucose eingesetzt wird.

14. Verfahren nach einem der Ansprüche 1 bis 13, **dadurch gekennzeichnet, daß** die Verfahrensstufen Hydrierung, Filtration, Entsalzung, Eindampfung und chromatographische Trennung als Einstrang-Prozeß durchgeführt werden.

15. Verfahren nach einem der Ansprüche 1 bis 14, **dadurch gekennzeichnet, daß** das bei den Eindampfungsprozessen anfallende Kondensat als Eluent in den Kreislauf der SSMB-Prozeßstufen zurückgeführt wird.

16. Verfahren nach einem der Ansprüche 1 bis 15, **dadurch gekennzeichnet, daß** zur Vermeidung großer Schütthöhen in den Säulen jede Säule in zwei gleich große Zellen unterteilt wird.

**Claims**

1. Method for the preparation of sorbitols by hydrogenation of glucose with a glucose content of 92 % to 96 % dry substance, deionization and evaporation up to 70 % dry substance content and subsequent chromatographic separation of the crude product arising in the form of a sugar alcohol mixture by the Sequential Simulated Moving Bed Method (SSMB) at a strongly acid cation exchanger, with each cycle consisting of a number of steps that correspond to the number of chromatographic zones, and each such step in turn being subdivided into four sequences, i.e. a first sequence 1 called "recycle", a second sequence 2 called "crude product flow", a third sequence 3 called "raffinate", and a fourth sequence 4 called "extract", and with sequences 2 and 4 within one single step being performed simultaneously and the raffinate fractions of the sequences 2 and 3 collected as one single aggregate fraction in the form of a commercial quality non-crystallizing sorbitol solution, and the columns' bed volumes changed to control separation results in terms of ion exchanger performance, composition of extract and raffinate as well as sorbitol content of the extract fraction so that, by varying the flow rate and/or the duration of one or more sequences are active, different extract fractions are obtained, i.e. sorbitol with a dry substance content of min. 98,5 % (pure sorbitol) or sorbitol with a dry substance content of min. 99,5 % (high-purity sorbitol), with the raffinate and extract fractions arising finally concentrated to solutions with up to 70 % dry substance content.

**2.** Method according to claim 1 **characterized in that**, by varying the duration of sequences 2 and 3 and/or the flow rates, the cells' bed volumes (within the zones) are adjusted as follows:

BV1 = 0,94 to 0,96, BV2 = 0,58 to 0,60, BV3 = 0,76 to 0,78, and BV4 = 0,38 to 0,40, so that the extract fraction obtained is composed of (a) sorbitol with a dry substance content of min. 98,5 %, total sugars with a dry substance content of max. 0,5 %, and residual glucose with a dry substance content of max. 0,06 %.

**3.** Method according to claim 1 **characterized in that**, by varying the duration of sequences 2 and 3 and/or the flow rates, the cells' bed volumes (within the zones) are adjusted as follows:

BV1 = 0,97 to 0,99, BV2 = 0,62 to 0,64, BV3 = 0,78 to 0,81, and BV4 = 0,38 to 0,40, so that the extract fraction obtained is composed of (b) high-purity sorbitol with a dry substance content of min. 99,5 %, total sugars with a dry substance content of max. 0,15 %, and residual glucose with a dry substance content of max. 0,02 %.

**4.** Method according to one of the claims 1 to 3 **characterized in that**, in order to switch the extract fraction quality from pure sorbitol to high-purity sorbitol, the duration of sequence 2 "crude product flow" is active is extended and the duration of sequence 3 "raffinate" is active is shortened, whereat, in order to switch the extract fraction quality from high-purity sorbitol to pure sorbitol, the duration of sequence 2 "crude product flow" is active is shortened and the duration of sequence 3 "raffinate" is active is extended, without changing any other process parameters.

**5.** Method according to claim 4 **characterized in that**, in order to switch the extract fraction type (a) to type (b), the duration of sequence 2 is active is extended by approx. 11 % and the duration of sequence 3 is active is shortened by approx. 15 %, whereat, in order to switch the extract fraction type (b) to type (a), the duration of sequence 2 is active is shortened by approx. 11 % and the duration of sequence 3 is active is extended by approx. 15 %.

**6.** Method according to one of the claims 1 to 5 **characterized in that** water in a quantity of 5 or 6 liter per kg dry substance is added as eluant.

**7.** Method according to one of the claims 1 to 6 **characterized in that** the chromatographic separation is performed at a temperature of up to 90 °C.

**8.** Method according to claim 7 **characterized in that**, maintaining the temperature at a level above 80 °C, the input comprises a crude product with 65 % to 70 % dry substance content and flow rates of up to 7 metres per hour are achieved.

**9.** Method according to one of the claims 1 to 8 **characterized in that** the crude product input has a dry substance content of 62 % to 70 %, preferably 65 % to 67 %.

**10.** Method according to one of the claims 1 to 9 **characterized in that** the pressure drop is maintained to not exceed 1,5 bar per metre when changing over from sequence 2 "crude product flow" to sequence 3 "raffinate".

**11.** Method according to one of the claims 1 to 10 **characterized in that** the sorbitol retention rate, i.e. the sorbitol percentile in the extract in relation to the sorbitol percentile in the crude product, is 75 % to 83 %.

**12.** Method according to one of the claims 1 to 11 **characterized in that** a daily specific performance, i.e. in relation to the input quantity of ion exchanger, of up to 2200 grammes of dry substance per liter of ion exchanger is achieved.

**13.** Method according to one of the claims 1 to 12 **characterized in that** the input crude product is a glucose gained directly from the starch hydrolysis process without any chromatographic or other enriching method being employed.

14. Method according to one of the claims 1 to 13 **characterized in that** the process steps hydrogenation, filtration, deionization, evaporation and chromatographic separation are performed as a single-strand method.

15. Method according to one of the claims 1 to 14 **characterized in that** the condensate arising during concentrating steps is recycled into the SSMB process stages to be used as eluant.

16. Method according to one of the claims 1 to 15 **characterized in that** each column is subdivided into two cells of equal size in order to avoid excessive dumping heights inside the columns.

**Revendications**

1. Procédé pour la préparation de sorbitols par hydrogénation de glucose présentant une teneur en glucose comprise entre 92 et 96 % de matière sèche (MS), par déminéralisation et réduction jusqu'à 70 % MS ainsi qu'en phase finale par séparation chromatographique du mélange d'alcool de sucre obtenu sous forme de produit brut d'après le procédé du lit mobile simulé séquentiel SSMB (Sequential Simulated Moving Bed) sur une résine échangeuse de cations fortement acide, chaque cycle se composant d'un nombre d'étapes correspondant au nombre de zones chromatographiques et chaque étape étant subdivisée en quatre séquences, en une première séquence 1 "circuit", une deuxième séquence 2 "entrée du produit brut", une troisième séquence 3 "raffinat" et une quatrième séquence 4 "extrait", et les séquences 2 et 4 étant réalisées simultanément au cours d'une même étape et les fractions Raffinat des séquences 2 et 3 étant récupérées en tant que fraction totale sous forme de solution de sorbitol non cristallisable, disponible dans le commerce, et les volumes de lit des colonnes étant modifiés pour influencer les résultats de la séparation par rapport au rendement de la résine, de la composition de l'extrait et du raffinat et de la teneur en sorbitol de la fraction Extrait, en modifiant la durée d'une ou de plusieurs séquences et/ou du débit, différentes fractions Extrait étant obtenues sous forme de sorbitol présentant une teneur en sorbitol d'au moins 98,5 % MS (sorbitol pur) ou sous forme de sorbitol présentant une teneur en sorbitol d'au moins 99,5 % MS (sorbitol de très grande pureté), et finalement les fractions Raffinat et Extrait qui se sont formées étant réduites en solutions présentant jusqu'à 70 % MS.

2. Procédé selon la revendication 1, **caractérisé en ce qu'**en modifiant la durée des séquences 2 et 3 et/ou des débits, les volumes de lit (BV) des cellules (contenues dans les zones) peuvent être ramenés aux valeurs suivantes :

   BV1 = 0,94 - 0,96, BV2 = 0,58 - 0,60, BV3 = 0,76 - 0,78 et BV4 = 0,38 - 0,40, et sous forme de fraction Extrait a) on obtient du sorbitol présentant une teneur en sorbitol d'au moins 98,5 % MS, une teneur totale en sucres maximale de 0,5 % MS et une teneur en glucose restant maximale de 0,06 % MS.

3. Procédé selon la revendication 1, **caractérisé en ce qu'**en modifiant la durée des séquences 2 et 3 et/ou des débits, les volumes de lit (BV) des cellules (contenues dans les zones) peuvent être ramenés aux valeurs suivantes :

   BV1 = 0,97 - 0,99, BV2 = 0,62 - 0,64, BV3 = 0,78 - 0,81 et BV4 = 0,38 - 0,40, et sous forme de fraction Extrait b) on obtient du sorbitol de très grande pureté présentant une teneur en sorbitol d'au moins 99,5 % MS, une teneur totale en sucres maximale de 0,15 % MS et une teneur en glucose restant maximale de 0,02 % MS.

4. Procédé selon l'une quelconque des revendications 1 à 3, **caractérisé en ce que**, pour changer la qualité de la fraction Extrait de sorbitol pur en sorbitol de très grande pureté, la durée de la séquence 2 "entrée du produit brut" est augmentée et la durée de la séquence 3 raffinat" est réduite et, pour retransformer le sorbitol de très grande pureté en sorbitol pur, la durée de la séquence 2 "entrée du produit brut" est réduite et la durée de la séquence 3 "raffinat" est augmentée sans modifier les autres paramètres du procédé.

5. Procédé selon la revendication 4, **caractérisé en ce que** pour le passage de la fraction Extrait a) à la fraction Extrait b), la durée de la séquence 2 est augmentée d'env. 11 % et celle de la séquence 3 est réduite d'env. 15 % et, pour le passage de la fraction Extrait b) à la fraction Extrait a), la durée de la séquence 2 est réduite d'env. 11 % et celle de la séquence 3 est augmentée d'env. 15 %.

6. Procédé selon l'une quelconque des revendications 1 à 5, **caractérisé en ce que** l'on utilise de l'eau comme éluant dans une quantité comprise entre 5 et 6 l/kg de matière sèche.

**7.** Procédé selon l'une quelconque des revendications 1 à 6, **caractérisé en ce que** la séparation chromatographique est réalisée à une température allant jusqu'à 90 °C.

**8.** Procédé selon la revendication 7, **caractérisé en ce qu'**en conservant des températures supérieures à 80 °C, on utilise un produit brut présentant une concentration MS comprise entre 65 et 70 % et on obtient des vitesses d'écoulement allant jusqu'à 7 m/h.

**9.** Procédé selon l'une quelconque des revendications 1 à 8, **caractérisé en ce que** le produit brut utilisé présente une concentration MS comprise entre 62 et 70 %, de préférence 65 % et 67 %.

**10.** Procédé selon l'une quelconque des revendications 1 à 9, **caractérisé en ce que**, pour le passage de la séquence 2 "entrée du produit brut" à la séquence 3 "raffinat", on conserve une perte de pression maximale de 1,5 bar/m.

**11.** Procédé selon l'une quelconque des revendications 1 à 10, **caractérisé en ce que** le sorbitol récupéré, c. à. d. la part de sorbitol contenue dans l'extrait par rapport à la part de sorbitol contenue dans le produit brut, est compris entre 75 et 83 %.

**12.** Procédé selon l'une quelconque des revendications 1 à 11, **caractérisé en ce que** l'on obtient une capacité se rapportant à la quantité de résine allant jusqu'à 2200 g de matière sèche/l de résine jour.

**13.** Procédé selon l'une quelconque des revendications 1 à 12, **caractérisé en ce que** l'on utilise comme produit brut un glucose extrait directement de l'hydrolyse de l'amidon et sans enrichissement par chromatographie ou autre.

**14.** Procédé selon l'une quelconque des revendications 1 à 13, **caractérisé en ce que** les étapes de procédés d'hydrogénation, filtration, déminéralisation, réduction et séparation chromatographique sont réalisées en mode uniligne.

**15.** Procédé selon l'une quelconque des revendications 1 à 14, **caractérisé en ce que** le condensat qui s'est formé au cours des processus de réduction est réintroduit sous forme d'éluant dans le circuit des étapes du procédé SSMB.

**16.** Procédé selon l'une quelconque des revendications 1 à 15, **caractérisé en ce que**, pour éviter des hauteurs de déversement importantes dans les colonnes, chaque colonne est subdivisée en deux cellules de même taille.

a) Schritt 1:

Wasserzufluß               Rohproduktzufluß

| Z1 | Z2 | Z3 | Z4 |

Kreislauf

Extrakt               Raffinat

b) Schritt 2:

Wasserzufluß               Rohproduktzufluß

| Z4 | Z1 | Z2 | Z3 |

Kreislauf

Extrakt               Raffinat

**Abbildung 1**

a) Schritt 1:

Sequenz 1:
"Kreislauf"

Z1    Z2    Z3    Z4

Produktzufluß

Sequenz 2:
"Rohproduktzufluß"

Z1    Z2    Z3    Z4

Raffinat

Wasser

Sequenz 3:
"Raffinat"

Z1    Z2    Z3    Z4

Raffinat

Wasser

Sequenz 4:
"Extrakt"

Z1    Z2    Z3    Z4

**Abbildung 2**

Extrakt